# EUROPEAN PATENT APPLICATION

(11) **EP 3 366 286 A1**
(43) Date of publication of application: **29.08.2018**
(21) Application number: 17157486.6
(22) Date of filing: 22.02.2017
(51) Int. Cl.: A61K 31/335, A61K 31/44, A61K 31/499, A61K 31/517, A61K 31/55, A61K 31/5513, A61K 31/5517, A61P 31/00

(54) **COMPOUNDS FOR TREATING SEPSIS**

(71) Applicant: Keller, Johannes, 13351 Berlin (DE)
(72) Inventor: Keller, Johannes, 13351 Berlin (DE); Amling, Michael, 22605 Hamburg (DE)
(74) Representative: CH Kilger Anwaltspartnerschaft mbB

(57) **Abstract**

The present invention relates to a compound for use in a method for treating a procalcitonin mediated systemic inflammation associated disease, particularly sepsis, wherein the compound is an antagonist or inhibitor of CRLR, RAMP1, RAMP2, RAMP3, or RCP.

## Description

The present invention relates to compounds for the treatment of systemic inflammation associated disease, particularly sepsis.

### Background

Sepsis represents the most common causes of death in non-cardiac intensive care units worldwide, as disease progression is associated with lethal systemic inflammation and multiple organ failure.

In sepsis, endotoxemia through the systemic release of lipopolysaccharide (LPS) from invading bacteria leads to a dysregulated immune response with dramatic alterations in systemic cytokine levels. This so called cytokine storm is accompanied by ubiquitous *Calca* expression and increased systemic procalcitonin (PCT). PCT is encoded by the *Calca* gene whose primary transcript is normally processed into calcitonin (CT) in the thyroid gland and αCgrp in the nervous system, respectively.

The above observation has resulted in the widespread clinical use of PCT as a specific and prognostic biomarker in sepsis. Several experimental studies have been conducted in the past to uncover potential biologic functions, however contradicting results have been reported fuelling persistent uncertainty regarding the effects of PCT in systemic inflammation. Moreover, the nature of the biologically relevant PCT receptor remains elusive, although its identification might enable the development of specific pharmacologic agents targeting PCT signalling in sepsis.

Present sepsis therapies base on the following four columns:
(1) removal of the underlying infection
(2) antimicrobial therapy
(3) liquid substitution and cardiovascular supportive medicaments, and
(4) organ replacement measures.

Although those measures significantly improve the sepsis therapy, the disease still is accompanied by high mortality and morbidity. Particularly, the above measures depend on a spontaneous and natural reoccurrence of a balanced immune system, which is in many cases to late.

Accordingly, there is still a high demand in means and methods for the treatment of sepsis.

### Description of the invention

Based on the above background, it is the objective of the present invention to provide novel, safe and efficient means for the treatment of sepsis. This objective is attained by a compound having the features of claim 1, a pharmaceutical composition having the features of claim 7, a dosage form having the features of claim 9, and methods having the features of claim 11 and 12 respectively.

According thereto, a first aspect of the invention relates to a compound for the use in a method for preventing or treating a procalcitonin mediated systemic inflammation associated disease, wherein the compound is an antagonist or inhibitor of CRLR (calcitonin-receptor-like receptor, Uniprot Q16602), RAMP1 (receptor activity modifying protein type 1, Uniprot O60894), RAMP2 (Uniprot O60895), RAMP3 (Uniprot O60896), or RCP (receptor component protein, Uniprot 075575).

The term "antagonist" is used in its meaning known in the art. It particularly refers to compound that inhibits or decreases the binding of procalcitonin to the calcitonin-receptor-like receptor and/or the signal transduction of calcitonin-receptor-like receptor triggered by the binding of procalcitonin, particularly by binding to the calcitonin-receptor-like receptor without provoking a biological response.

The term "inhibitor" is used in its meaning known in the art.

The term "procalcitonin mediated systemic inflammation associated disease" in the context of the present specification particularly refers to a medical condition, which is caused by pathophysiological effect of procalcitonin. Non limiting examples for procalcitonin mediated systemic inflammation associated diseases include sepsis, polytrauma, systemic inflammatory response syndrome, burn, acute cardiogenic shock, or acute respiratory distress syndrome.

The present invention is characterized by the following advantages:
- The compound of the invention directly modulates the misregulation of the immune system, thereby representing a causal therapy for procalcitonin mediated diseases such as sepsis.
- All degradation products of PCT being active at CRLR are inhibited by the compound of the invention.
- The compound of the invention may be applied by all clinical relevant and available application forms.
- The compound of the invention may be also applied for prophylaxis.
- The compound of the invention is characterized by a low side effect.
- The compound of the invention may be applied to human and animal patients.

In certain embodiments, the procalcitonin mediated systemic inflammation associated disease is sepsis.

In certain embodiments, the procalcitonin mediated disease is selected from polytrauma, systemic inflammatory response syndrome, acute cardiogenic shock, burn, or acute respiratory distress syndrome.

In certain embodiments, the compound is selected from the group comprised of:
- N-((1*R*)-2-(((1*S*)-5-amino-1-((4-(pyridin-4-yl)piperazin-1-yl)carbonyl)pentyl)amino)-1-(3,5-dibromo-4-hydroxybenzyl)-2-oxoethyl)-4-(2-oxo-1,4-dihydroquinazolin-3(2H)-yl)piperidine-1-carboxamide, also referred to as olcegepant or BIBN4096BS (Boehringer Ingelheim): 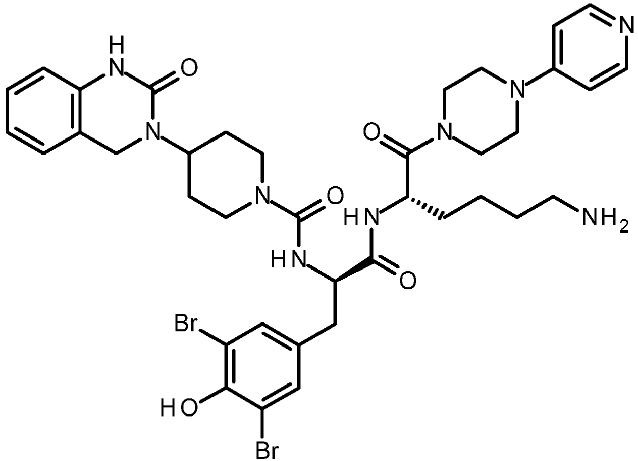
- N-((3R,6S)-6-(2,3-difluorophenyl)-2-oxo-1-(2,2,2-trifluoroethyl)hexahydro-1 H-azepin-3-yl)-4-(2-oxo-2,3-dihydro-1H-imidazo[4,5-b]pyridin-1-yl)piperidine-1-carboxamide, also referred to as telcagepant or MK-0974 (Merck): 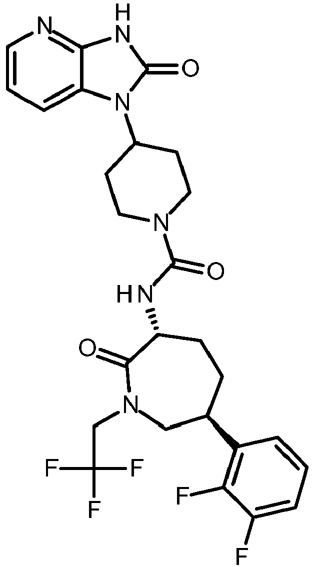
- 2-((8*R*)-8-(3,5-difluorophenyl)-10-oxo-6,9-diazaspiro(4.5)dec-9-yl)-N-((2R)-2'-oxo-1,1',2',3-tetrahydrospiro(indene-2,3'-pyrrolo[2,3-b]pyridin)-5-yl)acetamide also referred to as MK-3207 (Merck): 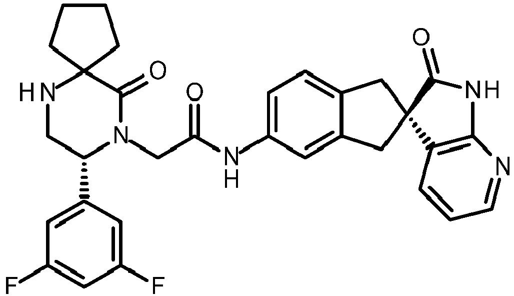
- CGRP₈₋₃₇ (calcitonin gene-related peptide residues 8 to 37);
- a ligand specifically reactive to any one of CRLR, RAMP1, RAMP2, RAMP3 or RCP;
- a modulator of expression of a gene encoding any one of CRLR, RAMP1, RAMP2, RAMP3 or RCP;
- a hydroxy pyridin carboxamide CRLR antagonist;
- an oxepino[3,4-e]indazol-8-one CRLR antagonist;
- an azepino[3,4-e]indazol-8-one CRLR antagonist; or
- an azepine CRLR antagonist.

The term "ligand specifically reactive to CRLR, RAMP1, RAMP2, RAMP3 or RCP" in the context of the present specification particularly refers to a ligand that is able to bind to CRLR, RAMP1, RAMP2, RAMP3 or RCP with a dissociation constant of 10⁻⁷, 10⁻⁸ or 10⁻⁹ mol/l or less.

A ligand according to the invention may be any molecule that binds to a target molecule or analyte with high affinity and specificity. Such a ligand may be an antibody, an antibody fragment, an antibody-like molecule or a nucleic acid aptamer molecule of 10 to 75 nucleotides in length, any of which binds to the target molecule.

An antibody fragment may be a Fab fragment, which is the antigen-binding fragment of an antibody, or a single-chain variable fragment, which is a fusion protein of the variable regions of the heavy and the light chain of an antibody connected by a peptide linker. An antibody-like molecule may be a repeat protein, such as a designed ankyrin repeat protein (Molecular Partners, Zurich).

Suitable ligands according to the above aspect of the invention may also be developed by methods such as phage display, ribosome display or SELEX, wherein polypeptide or oligonucleotides are selected due to their binding affinity to a target of interest. Additionally, the binding affinity of an identified ligand may be improved by cycles of evolution of the amino acid sequence or nucleotide sequence, and selection of the evolved inhibitors may be effected based on the required affinity.

In certain embodiments, the ligand specifically reactive to any on of CRLR, RAMP1, RAMP2, RAMP3 or RCP is an antibody, anti-body fragment or antibody-like, particularly a monoclonal antibody.

In certain embodiments, the modulator of expression of a gene encoding any one of CRLR, RAMP1, RAMP2, RAMP3 or RCP is a nucleic acid molecule or a precursor thereof comprising a sequence tract complementary to an mRNA molecule encoding any one of CRLR, RAMP1, RAMP2, RAMP3 or RCP, wherein the nucleic acid molecule is particularly a single-stranded or double stranded RNA or DNA molecule.

The art of silencing or "knocking down" genes, by degradation of mRNA or other effects, is well known. A multitude of mechanisms of action for, and definitions of, such RNAdriven interference with gene expression has been discovered and adapted to practical use during the last decade. Examples of technologies identified are siRNA, miRNA, shRNA, shmiRNA, or dsRNA. A comprehensive overview of this field can be found in Perrimon et al, Cold Spring Harbour Perspectives in Biology, 2010, 2, a003640.

Likewise, the modulator may be an expression vector comprising an RNA polymerase promoter sequence operable in a mammalian cell, and an expressed sequence encoding the above named nucleic acid molecule or a precursor thereof. Such expression vector leads to production of an interfering RNA within the cell. Methods for making and using such expression vectors are known in the art.

Alternatively, the modulator may be a single-stranded or double-stranded antisense ribonucleic or deoxyribonucleic acid, 30 comprising sequences complementary to a regulatory region of a gene encoding a member of the group comprised of CRLR, RAMP1, RAMP2, RAMP3 or RCP.

Such antisense molecules may be 12-50 nucleotides in length, preferably 18-30 nucleotides, and have a sequence comprised in an exon or intron of any of CRLR, RAMP1, RAMP2, RAMP3 or RCP. Moreover, antisense molecules comprising a sequence forming part of the promoter sequence regulating transcription of any of CRLR, RAMP1, RAMP2, RAMP3 or RCP, and binding to one of the strands of the promoter region, may be used. Finally, antisense molecules binding in the 3' UTR -non-translated regions of any of the subunits of CRLR, RAMP1, RAMP2, RAMP3 or RCP are contemplated.

Non-limiting examples for hydroxy pyridine carboxamide CRLR antagonists include:
- 4-bromo-N-(3,5-difluorobenzyl)-6-(1,1-dioxido-1,2-thiazinan-2-yl)-3-hydroxypyridine-2-carboxamide;
- 4-bromo-N-(3-fluorobenzyl)-6-(1,1-dioxido-1,2-thiazinan-2-yl)-3-hydroxypyridine-2-carboxamide; 4-bromo-N-(t-butyl-acetate)-6-(1,1-dioxido-1,2-thiazinan-2-yl)-3-hydroxypyridine-2-carboxamide;
- 4-bromo-N-((cyclobutylcarbamoyl)methyl)-6-(1,1-dioxido-1,2-thiazinan-2-yl)-3-hydroxypyridine-2-carboxamide; 4-bromo-N-((cyclohexylcarbamoyl)methyl)-6-(1,1-dioxido-1,2-thiazinan-2-yl)-3-hydroxypyridine-2-carboxamide;
- 4-bromo-N-((adamantylcarbamoyl)methyl)-6-(1,1-dioxido-1,2-thiazinan-2-yl)-3-hydroxypyridine-2-carboxamide;
- 4-bromo-N-((m-diphenylcarbamoyl)methyl)-6-(1,1-dioxido-1,2-thiazinan-2-yl)-3-hydroxypyridine-2-carboxamide; N-(3,5-difluorobenzyl)-6-(1,1-dioxido-1,2-thiazinan-2-yl)-8-hydroxynaphtyridine-7-carboxamide; N-(3-iodobenzyl)-6-(1,1-dioxido-1,2-thiazinan-2-yl)-8-hydroxynaphtyridine-7-carboxamide;
- N-(3,5-dichlorobenzyl)-6-(1,1-dioxido-1,2-thiazinan-2-yl)-8-hydroxynaphtyridine-7-carboxamide; methyl 2-{[3,5-difluorobenzyl)amino]carbonyl}-6-(1,1-dioxido-1,2-thiazinan-2-yl)-3-hydroxyisonicotinate; and
- methyl 2-({[t-butoxycarbonyl)methyl]amino}carbonyl)-6-(1,1-dioxido-1,2-thiazinan-2-yl)-3-hydroxyisonicotinate.

Non-limiting examples for oxepino[3,4-e]indazol-8-one and azepino[3,4-e]indazol-8-one CRLR antagonists include:
- (*S*)-4-bromo-7-(2-oxo-2-(4-(2-oxo-1,2-dihydroquinolin-3-yl)piperidin-1-yl)ethyl)-6,7-dihydro-3H-oxepino[3,4-e]indazol-8(10H)-one;
- (*S*)-1,4-dibromo-7-(2-oxo-2-(4-(2-oxo-1,2-dihydroquinolin-3-yl)piperidin-1-yl)ethyl)-9-(2,2,2-trifluoroethyl)-6,7,9,10-tetrahydroazepino[3,4-e]indazol-8(3H)-one;
- (*S*)-1,4-dibromo-7-(2-(4-(8-fluoro-2-oxo-1,2-dihydroquinolin-3-yl)piperidin-1-yl)-2-oxoethyl)-9-(2,2,2-trifluoroethyl)-6,7,9,10-tetrahydroazepino[3,4-e]indazol-8(3H)-one.

Non-limiting examples for azepine CRLR antagonists include:
- N-[(3*R*,7*R*)-1-(cyclopropylmethyl)-2-oxo-7-phenylazepan-3-yl]-4-(2-oxo-1,4-dihydroquinazolin-3 (2H)-yl)piperidine-1-carboxamide;
- *tert*-butyl [(3*R*,7*R*)-2-oxo-3-({[4-(2-oxo-1,4-dihydroquinazolin-3 (2H)-yl)piperidin-1-yllcarbonyl}amino)-7-phenylazepan-1-yl acetate;
- *tert*-butyl [(3*R*,7*R*)-2-oxo-3-({[4-(2-oxo-1,2,4,5-tetrahydro-3H-1,3-benzodiazepin-3-yl)piperidin-1-yllcarbonyl}amino)-7-phenylazepan-1-yl]acetate;
- N-R (3*R*,6*S*)-1-(cyclopropylmethyl)-2-oxo-6-phenylazepan-3-yl]-4-(2-oxo-1,4-dihydroquinazolin-3(2H)-yl) piperidine-1-carboxamide;
- N-R (3*S*,6*R*)-1-(cyclopropylmethyl)-2-oxo-6-phenylazepan-3-yl]-4-(2-oxo-1,4-dihydroquinazolin-3(2H)-yl) piperidine-1-carboxamide;
- cis-N-1-(cyclopropylmethyl)-2-oxo-6-phenylazepan-3-yl]-4-(2-oxo-1,4-dihydroquinazolin-3(2H)-yl) piperidine-1-carboxamide;
- N-R(3*R*,6*S*)-1-(cyclopropylmethyl)-2-oxo-6-phenylazepan-3-yll-4-(2-oxo-1,2,4,5-tetrahydro-3H-13-benzodiazepin-3-yl) piperidine-1-carboxamide;
- 4-(4-chloro-2-oxo-2,3-dihydro-1 H-benzimidazol-1-yl)-N-f (3R,6S)-1-(2-methoxyethyl)-2-oxo-6-phenylazepan-3-yllpiperidine-1-carboxamide;
- N-f (3R,6S)-1-(2-methoxyethyl)-2-oxo-6-phenylazepan-3-yll-4-(4-methyl-2-oxo-2,3-dihydro-1H-benzimidazol-1-yl) piperidine-1-carboxamide;
- N-r (2S,6R)-4-(cyclopropylmethyl)-5-oxo-2-phenyl-14-oxazepan-6-yll-4-(2-oxo-1,2,4,5-tetrahydro-3H-1,3-benzodiazepin-3-yl) piperidine-1-carboxamide;
- N-(2*S*,6*R*)-4-(cyclopropylmethyl)-5-oxo-2-phenyl-1,4-oxazepan-6-yll-4-(2-oxo-2,3-dihydro-1H-benzimidazol-1-yl) piperidine-1-carboxamide;
- N-r (2*S*,6*R*)-4-(cyclopropylmethyl)-5-oxo-2-phenyl-1,4-oxazepan-6-yll-4-(6-fluoro-2-oxo-2,3-dihydro-1H-benzimidazol-1-yl) piperidine-1-carboxamide;
- N-f(2S*6R)-4-(cyclopropylmethyl)-5-oxo-2-phenyl-1,4-oxazepan-6-yll-4-(2-oxo-1,2-dihydroquinolin-3-yl) piperidine-1-carboxamide;
- N-r (2S, 6R and 2R,6S)-4-(cyclopropylmethyl)-5-oxo-2-phenyl-1,4-oxazepan-6-yll-4-(2-oxo-1,4-dihydroquinazolin-3(2H)-yl) piperidine-1-carboxamide;
- 19-(2R,6S and 2*R*,6*R*)-4-(cyclopropylmethyl)-5-oxo-2-phenyl-1,4-oxazepan-6-yll-4-(2-oxo-1,4-dihydroquinazolin-3(2H)-yl) piperidine-1-carboxamide;
- cis N-r (3S,6S and 3R,6R)-1-(cyclopropylmethyl)-7-oxo-3-phenyl-1,4-diazepan-6-yll-4-(2-oxo-1,4-dihydroquinazolin-3 (2H)-yl) piperidine-1-carboxamide;
- N-[(3*R*)-1-(cyclopropylmethyl)-2-oxoazepan-3-yll-4-(2-oxo-1,4-dihydroquinazolin-3 (2H)-yl) piperidine-1-carboxamide;
- N-r (3*R*)-1-benzyl-2-oxoazepan-3-yll-4-(2-oxo-1,4-dihydroquinazolin-3(2H)-yl) piperidine-1-carboxamide;
- N-(1-benzyl-2-oxoazepan-3-yl)-4-(2-oxo-1,2,4,5-tetrahydro-3H-1,3-benzodiazepin-3-yl) piperidine-1-carboxamide;
- N-f (3*R*)-1-(4-hydroxybenzyl)-2-oxoazepan-3-yll-4-(2-oxo-1,4-dihydroquinazolin-3(2H)-yl) piperidine-1-carboxamide;
- N-f (3R)-1-(3-methoxybenzyl)-2-oxoazepan-3-yll-4-(2-oxo-1,4-dihydroquinazolin-3(2H)-yl) piperidine-1-carboxamide;
- N-r (3*R*)-1-(3-Hydroxybenzyl)-2-oxoazepan-3-yll-4-(2-oxo-1 4-dihydroquinazolin-3(2H)-yl) piperidine-1-carboxamide;
- N-[1-benzyl-3-(4-hydroxybenzyl)-2-oxoazepan-3-yl]-4-(2-oxo-1,4-dihydroquinazolin-3(2H)-yl) piperidine-1-carboxamide; and
- N-[3-(4-methoxybenzyl)-2-oxoazepan-3-yll-4-(2-oxo-2,3-dihydro-1H-benzimidazol-1-yl) piperidine-1-carboxamide.

Further non-limiting examples for suitable CRLR antagonists include small molecule as disclosed in: US 8,394,767, PCT/EP03/11762, PCT/EP03/11763, PCT/EP2004/000087, PCT/EP2005/003094, PCT/US03/16576, PCT/US2004/040721, PCT/US2003/038799, PCT/US2005/010330, PCT/GB99/03154, PCT/US2004/007226, PCT/US2004/007289, PCT/US2004/007686, PCT/US2004/007678, PCT/US2004/007715, PCT/US2004/011254, PCT/US2004/010851, PCT/US2004/011280, PCT/US2004/020206, PCT/US2004/021888, PCT/US2004/020209, PCT/US2005/002199, PCT/US2005/031713, PCT/US2005/031617, PCT/US2005/031712, PCT/US2005/032036, PCT/US2005/032041, PCT/US2005/032288, PCT/US2005/035654, US 20080139537, US 20080139591, US 20080125413, US 20080113966, US 20080096878, US 20080090806, US 20080070899, US 20080004304, US 20080004261, US 20070293470, US 20070287697, US 20070287696, US 20070265225, US 2007/0259850, US 20070225272, US 20070149503, US 20070149502, US 20070111982, US 20070049577, US 20060211712, US 20060194783, US 20060189600, US 20060189593, US 20060183700, US 20060173046, US 20060148790, US 20060148779, US 20060135511, US 2006/0094707, US 20050256098, US 20050215576, US 20040229861, US 20040204397, and US 20040063735.

Further non-limiting examples for suitable CRLR antagonists include peptides as disclosed in US 20020068814 and US 20080020978.

In certain embodiments, the compound of the invention is systemically administered.

In certain embodiments, the compound of the invention is locally administered provided that the compound of the invention is not locally administered at a local inflamed or infected site, particularly the site that primarily causes the systemic inflammation.

In certain embodiments, the compound of the invention is epicutaneously, subcutaneously, intramuscularly, intravenously, intraarterially, intraperitoneally, intraspinally, intracerebrally, peridurally, intradurally, intrapulmonally, rectally, vaginally, nasally, orally or sublingually administered, preferably orally, intravenously, intraperitoneally or subcutaneously.

In certain embodiments, the compound of the invention is administered to patient in a concentration in the range of 0.01 mg to 100 mg per kg body weight of the patient, preferably in the range of 1 mg to 10 mg per kg body weight of the patient.

In certain embodiments, the compound of the invention is administered once or multiple times daily or weekly, intermittently, or continuously e.g. by means of a pump system.

According to a further aspect of the invention, a pharmaceutical composition for use in a method for preventing or treating a procalcitonin mediated systemic inflammation associated disease, particularly sepsis, polytrauma, systemic inflammatory response syndrome, acute cardiogenic shock, burn, or acute respiratory distress syndrome, is provided, comprising the compound of the invention, and optionally a pharmaceutical acceptable carrier and/or excipient.

In certain embodiments, the pharmaceutical composition is formulated for epicutaneous, subcutaneous, intramuscular, intravenous, intraarterial, intraeritoneal, intraspinal, intracerebral, peridural, intradural, intrapulmonal, rectal, vaginal, nasal, oral or sublingual application.

In certain embodiments, the pharmaceutical composition is formulated as injection form or injection solution, ointment, cream, suspension patch, gel, tablet, powder, spray, syrup or inhalation aerosol.

An injection form of the pharmaceutical composition is preferred, particularly for intravenous, subcutaneous or intraperitoneal application. Particularly, solutions of a compound according to the first aspect or any one of the corresponding embodiments of the invention can be made up shortly before use as an injection form. Similarly, suspensions or dispersions, especially isotonic aqueous solutions, dispersions or suspensions may be employed.

The pharmaceutical compositions for oral administration also include hard capsules consisting of gelatin, and also soft, sealed capsules consisting of gelatin and a plasticizer, such as glycerol or sorbitol. The capsules may contain the active ingredient in the form of granules, or dissolved or suspended in suitable liquid excipients, such as in oils.

According to further aspect of the invention, a dosage form for use in a method for preventing treating a procalcitonin mediated systemic inflammation associated disease, particularly sepsis, polytrauma, systemic inflammatory response syndrome, acute cardiogenic shock, burn, or acute respiratory distress syndrome, is provided, comprising the compound of the invention, and optionally a pharmaceutical acceptable carrier and/or excipient.

In certain embodiments, the compound of the invention comprised within the dosage form is formulated as an oral formulation, nasal inhalant, injection form, suppository or topical formulation.

According to yet another aspect of the invention, method of manufacturing a medicament for preventing or treating a procalcitonin mediated systemic inflammation associated disease, particularly sepsis, polytrauma, systemic inflammatory response syndrome, acute cardiogenic shock, burn, or acute respiratory distress syndrome, is provided, comprising the use of the compound of the invention. Medicaments according to the invention are manufactured by methods known in the art, especially by conventional mixing, coating, granulating, dissolving or lyophilizing.

According to yet another aspect of the invention, a method for preventing or treating a procalcitonin mediated systemic inflammation associated disease, particularly sepsis, polytrauma, systemic inflammatory response syndrome, acute cardiogenic shock, burn, or acute respiratory distress syndrome, is provided, comprising administering the compound of the invention to a patient in need thereof, particularly said compound in a pharmaceutically effective amount.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Description of the figures

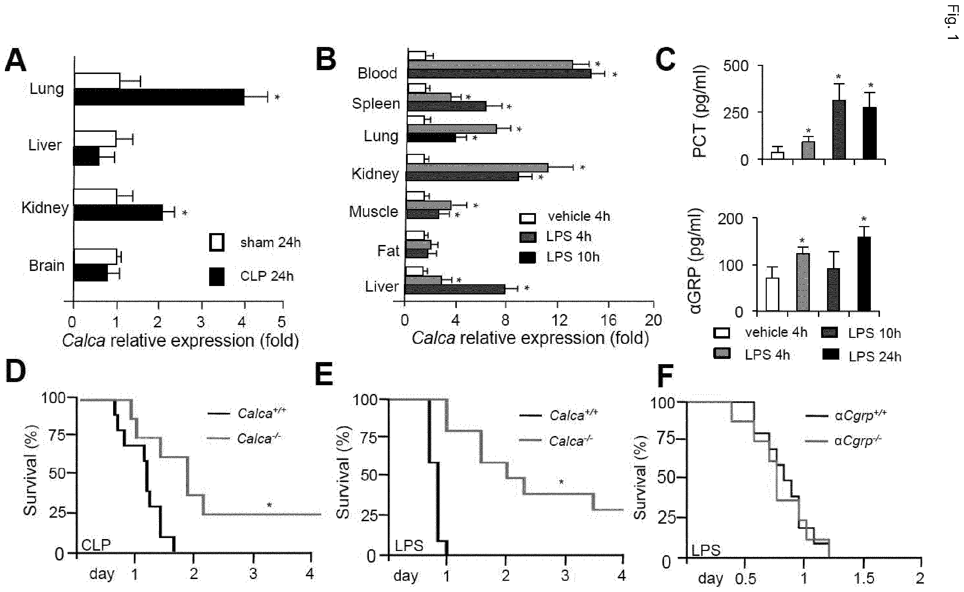

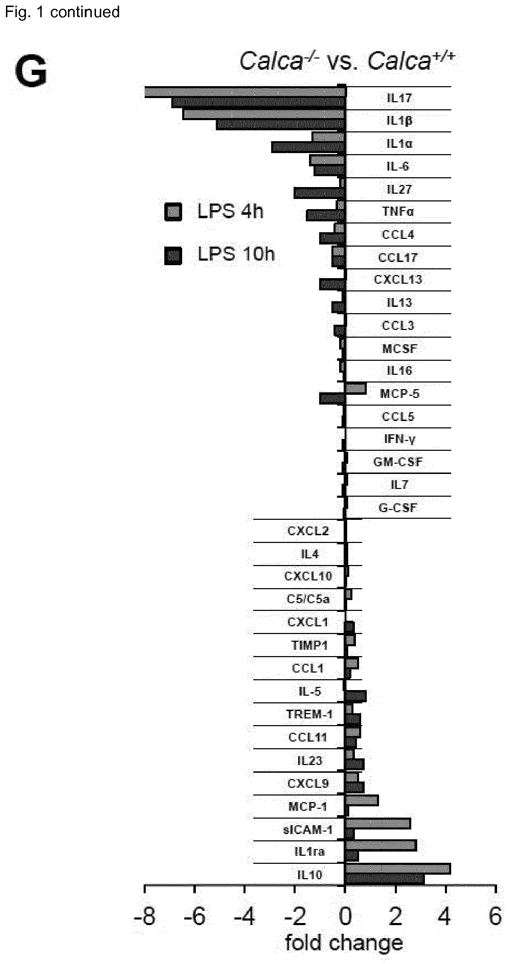

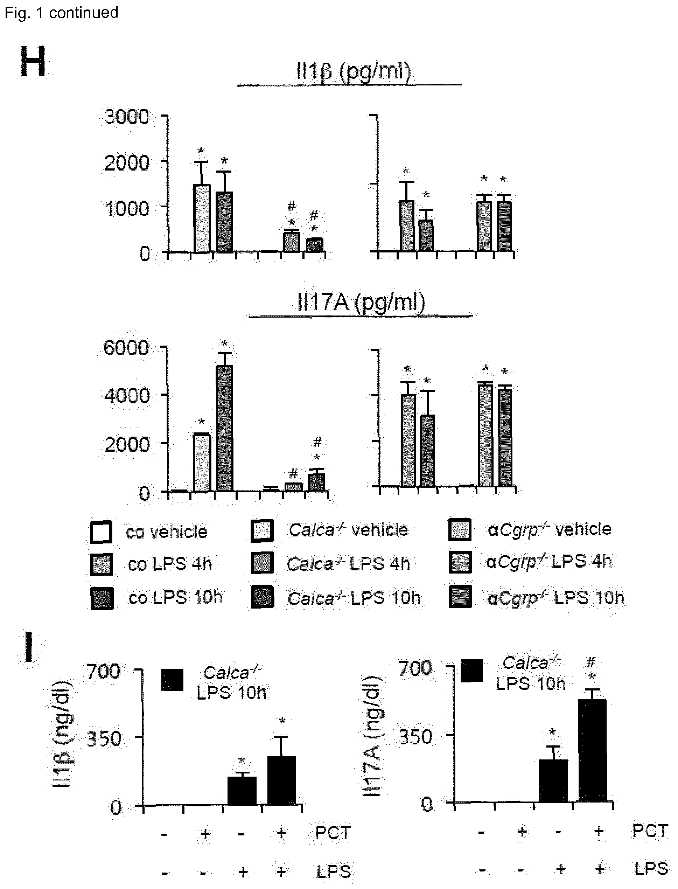

- Fig. 1: shows an increased survival of *Calca*-deficient mice in experimental septic shock. **A)** Relative expression of *Calca* in various organs derived from WT mice 24 h after CLP and **B)** 4 and 10 h after 10 mg/kg LPS i.p. n=4-6 mice per group. **C)** Serum concentrations of PCT and αCgrp 4, 10, and 24 h in WT mice after 10 mg/kg LPS i.p. n=4-6 mice per group. **D)** Survival of *Calca^{-l-}* mice and controls (*Calca*^{+/+}) subjected to CLP (n=8-9 mice per group). **E)** Survival *of Calca*^{*-*/*-*} or **F)** *αCgrp*^{*-*/*-*} mice and respective controls after 40 mg/kg LPS i.p. n=9-12 mice per group. For all experiments survival was monitored for 7 days. **G)** Protein profiling of serum cytokines in *Ca*/*ca*-deficient mice 4 and 10 h after 10 mg/kg LPS ip (pooled serum of n=4 per group). **H)** Confirmation of selected cytokines by ELISA in serum of *Calca*- and *αCgrp-*deficient mice after 10 mg/kg LPS. co=respective littermate controls. n=5 mice per group. **I)** Serum levels of II1β and II17a in *Ca*/*ca*-deficient mice 10 h after treatment with 10 mg/kg LPS, 100 g/kg PCT, or both i.p. (n=4). *p<0.05 indicates statistically significant differences compared to controls, #p<0.05 indicates statistically significant differences between treated groups.

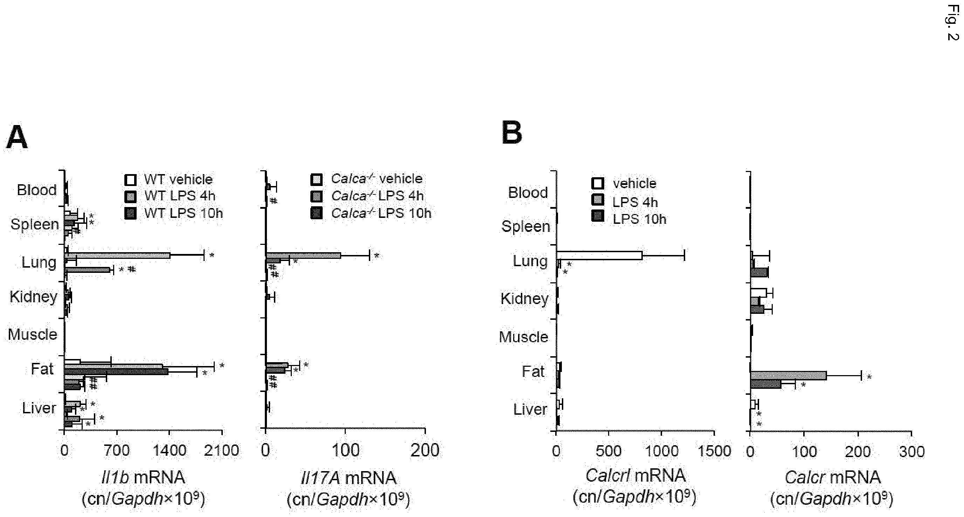

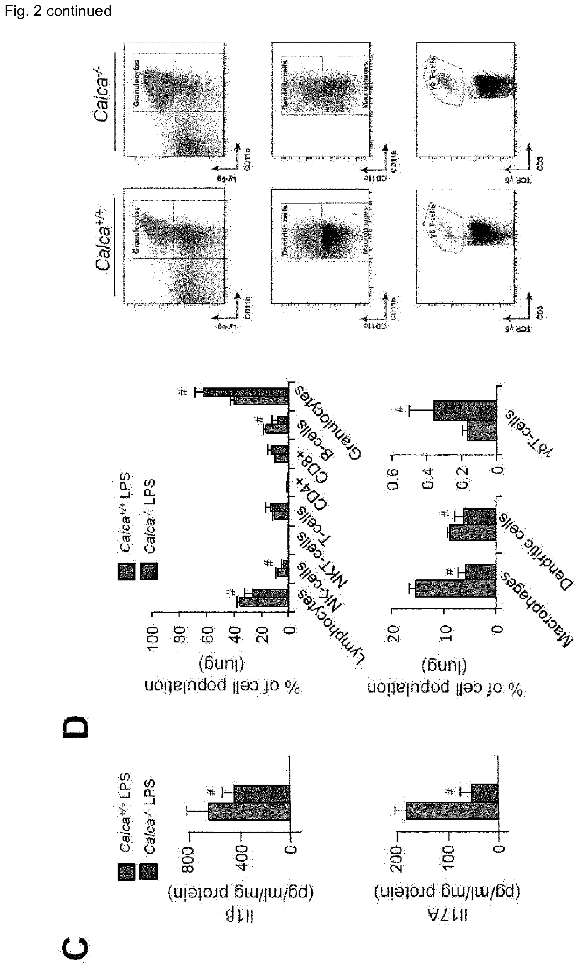

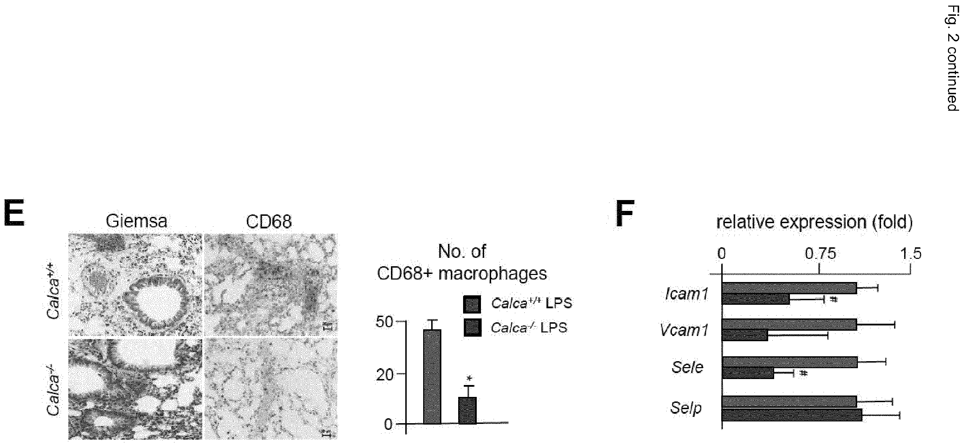

- Fig. 2: shows decreased pulmonary II17a and II1β levels accompanied by reduced macrophage influx in *Ca*/*ca*-deficient mice. **A)** qRT-PCR expression analyses of *II17*a and *II1b* in various organs of WT and *Calca*^{-/-} mice 4 and 10 h after 10 mg/kg LPS i.p. (n=4). **B)** Expression of putative PCT receptors in WT mice 4 and 10 h after 10 mg/kg LPS i.p. (n=4). **C)** ELISA of II17a and II1β levels in flushed lungs from *Ca*/*ca*-deficient mice 10 h after treatment with 10 mg/kg LPS i.p. (n=4). **D)** FACS expression analyses of indicated cell populations in flushed lung digests from *Calca*^{+/+} and *Calca*^{-/-} mice 10 h after 10 mg/kg LPS i.p. (n=4). **E)** Masson's trichrome staining and immunohistochemistry using a CD68-specific antibody of non-flushed lungs from *Calca*^{+/+} and *Calca*^{-/-} mice 10 h after 10 mg/kg LPS i.p. (n=4). Quantification of intravascular CD68 positive cells is displayed on the right. **F)** qRT-PCR analyses of indicated genes in lungs from the same groups. *p<0.05 indicates statistically significant differences compared to controls, #p<0.05 indicates statistically significant differences between treated groups.

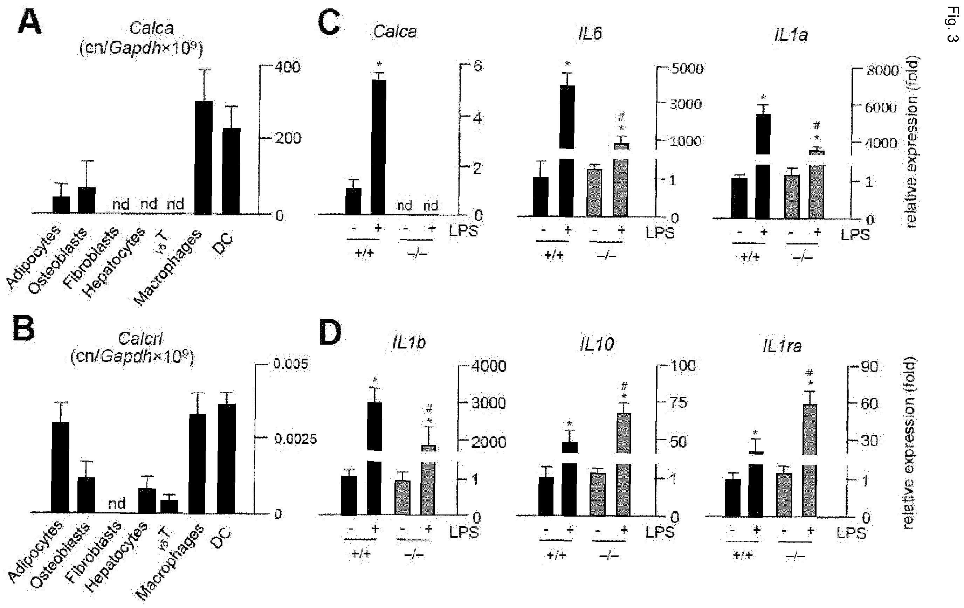

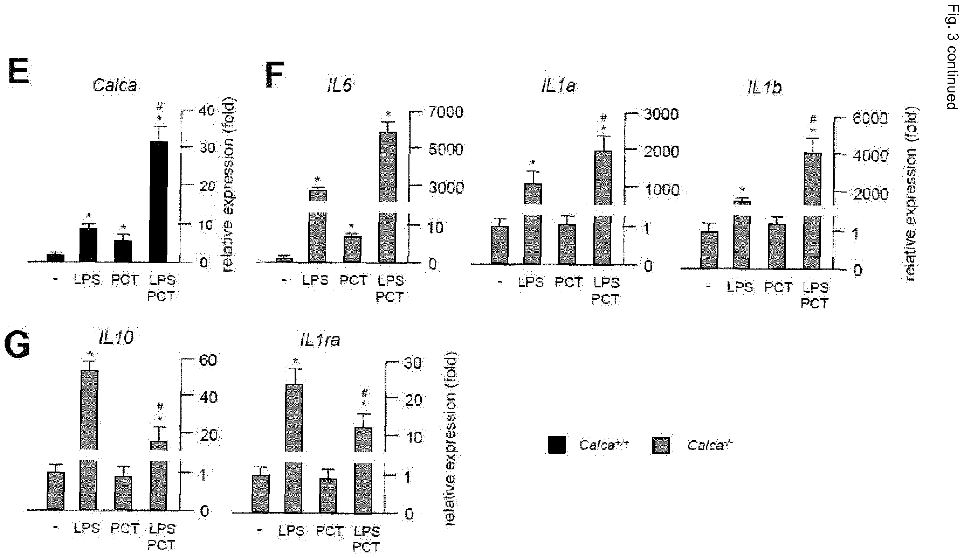

- Fig. 3: shows that PCT modulates LPS-induced stimulation of cytokine expression in macrophages. **A)** *Calca* expression in various primary cell types stimulated with 10 µg/ml LPS for 6 h (n=4 cultures) and **B)** *Calcrl* expression in various untreated primary cell types (bottom, n=4 cultures). **C**, **D)** Expression analyses of selected genes in bone-marrow derived macrophages of the indicated genotypes stimulated with 10 µg/ml LPS for 6 h (n=4 cultures). **E-G)** Expression analyses of the indicated genes in bone-marrow derived macrophages of the indicated genotypes stimulated with 10 µg/ml LPS and/or 200 ng/ml PCT for 6 h (n=4 cultures). *p<0.05 indicates statistically significant differences compared to controls, #p<0.05 indicates statistically significant differences between treated groups.

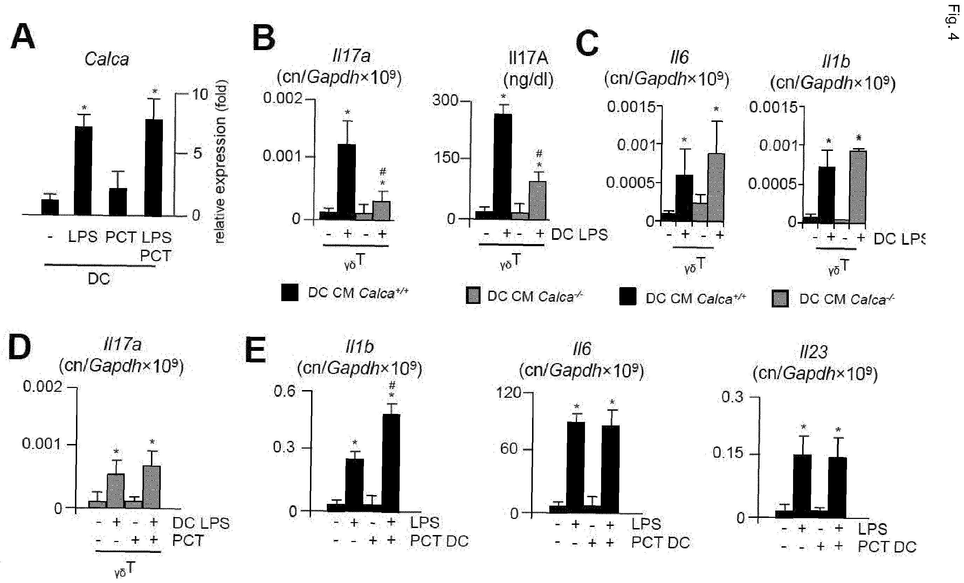

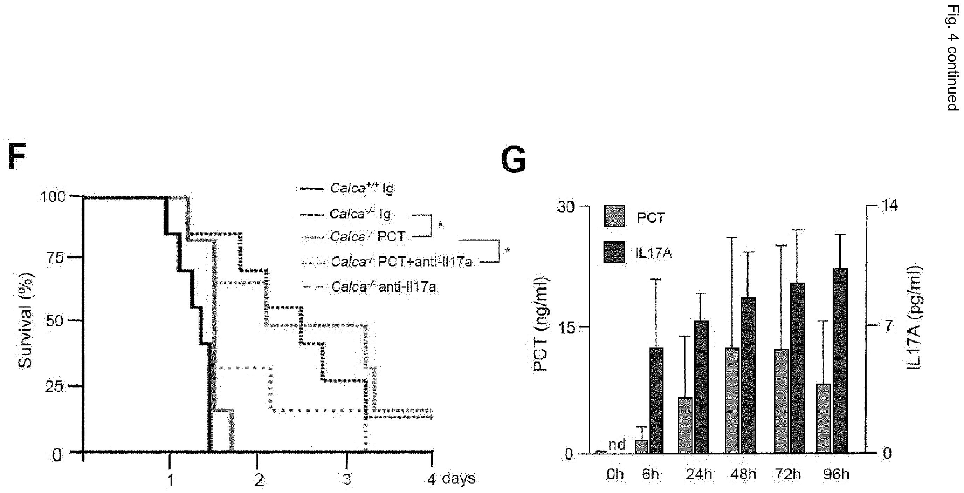

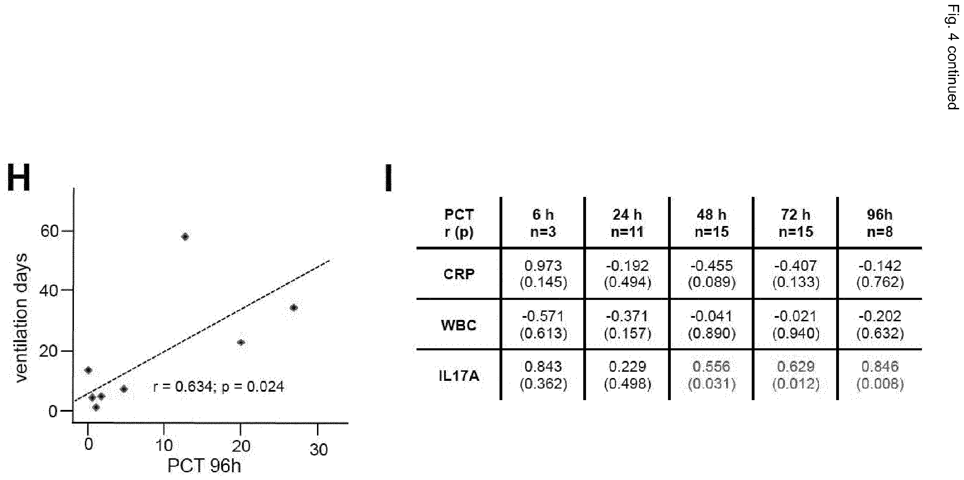

- Fig. 4: shows negative effect of PCT through II-17a on sepsis progression. **A)** Expression of *Calca* in dendritic cells (DC) stimulated with 10 µg/ml LPS and/or 200 ng/ml PCT for 6 h (n=3 cultures). **B)** *II17a* mRNA and protein levels in culture supernatants of WT γδT cells after stimulation for 24h with conditioned medium from DC of the indicated genotypes treated with vehicle (-) or 10 µg/ml LPS (+) for 24 h (n=3 cultures). **C)** Expression of the indicated genes in WT γδT cells receiving the same treatment. **D)** Expression of *II17a* in WT γδT cells stimulated with conditioned medium from *Calca*^{-/-}-DC treated with vehicle (DC LPS -) or LPS (DC LPS +) for 24 h, and substituted with vehicle (PCT -) or 200 ng/ml PCT (PCT +) before addition to γδT cell cultures (n=3 cultures). **E)** Expression of selected genes in γδT cell cultures receiving the same treatment regimen. *p<0.05 indicates statistically significant differences compared to controls, #p<0.05 indicates statistically significant differences between treated groups. **F)** Survival of mice of the indicated genotypes receiving i.p. injections of 40 mg/kg LPS and either non-specific immunoglobulins as control (Ig), 100 µg/kg PCT, 2.5 µg/kg anti-II-17A or both ip, as indicated (n=9-12 mice per group). *p<0.05 indicates statistically significant differences as indicated. **G)** PCT and IL17A levels in polytrauma patients subsequently developing sepsis at the indicated time points following admission. **H)** Dot-blot presentation of PCT levels 96h after admission and ventilation days in affected individuals. The Pearson correlation coefficient, respective p value and trend line is indicated within the blot. **I)** Pearson correlation between PCT and inflammatory markers (CRP=C reactive protein; WBC= white blood cells) at the indicated time points after admission.

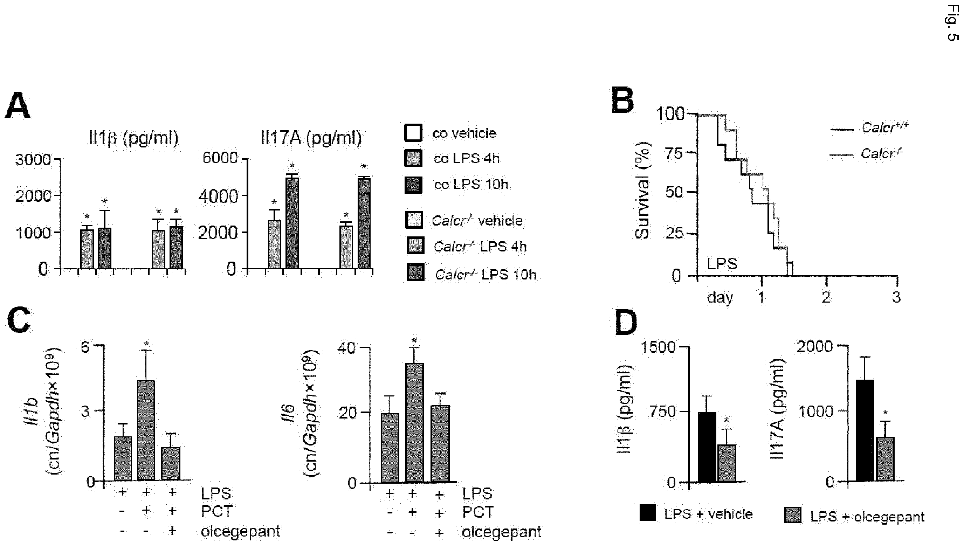

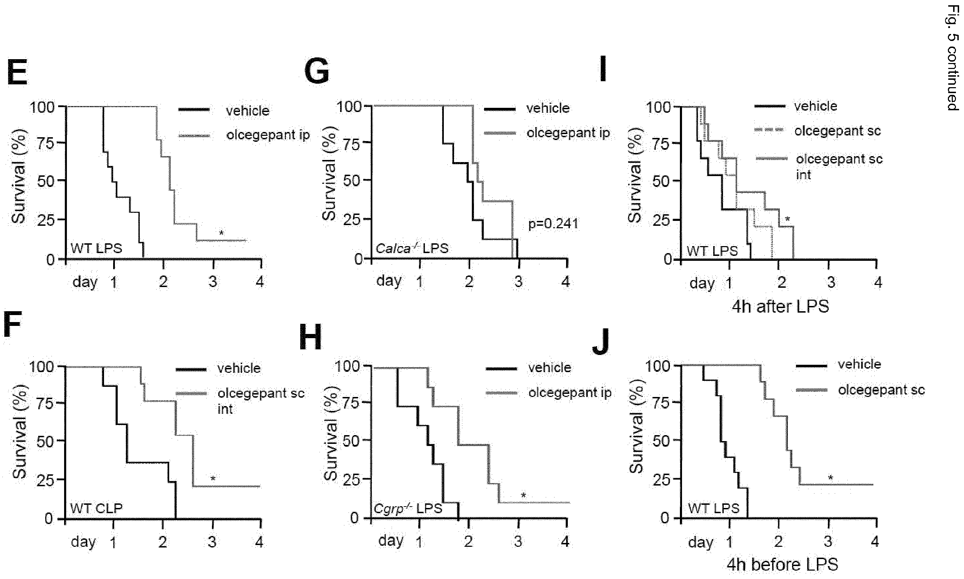

- Fig. 5: shows that antagonizing PCT at CRLR protects from lethal systemic inflammation. **A)** ELISA of selected cytokines in serum of *Calcr*-deficient mice and littermate controls (co) after 10 mg/kg LPS. n=5 mice per group. **B)** Survival of calcitonin receptor-(*Calcr*) deficient mice mice receiving ip injections of 40 mg/kg LPS. n=9-11 mice per group. **C)** mRNA levels of indicated cytokines in bone marrow-derived macrophages receiving the indicated treatment (n=3 cultures per group). **D)** Serum levels of II1β and II17A in WT mice 10 h after 10 mg/kg LPS and a single injection of either vehicle or 100 mg/kg olcegepant ip (n=4-6 mice per group). **E)** Survival of WT mice receiving ip injections of either vehicle or olcegepant (100 mg/kg) immediately before 40 mg/kg LPS ip (n=8-10 mice per group). **F)** Survival of WT mice subjected to CLP and treated subcutaneously with vehicle or intermittent (int) olcegepant (100 mg/kg) every 12h. **G)** Survival of *Ca*/*ca*-deficient mice receiving olcegepant (100 mg/kg ip) immediately before 40 mg/kg LPS (n=8-11 mice per group). **H)** Survival of *αCgrp-*deficient mice receiving the same treatment. **I)** Survival of WT mice subcutaneously receiving a single or intermittent (int) olcegepant injections (100 mg/kg) 4h after LPS challenge as indicated (n=9 mice per group). **J)** Survival of WT mice subcutaneously receiving olcegepant injections 4h before LPS (40 mg/kg ip, n=8-10 mice per group). For all experiments survival was monitored for 7 days. *p<0.05 indicates statistically significant differences.
- Fig. 6: shows serum concentrations of CT 4, 10, and 24 h in WT mice after 10 mg/kg LPS i.p. n=4-6 mice per group.
- Fig. 7: shows the confirmation of cytokine levels by ELISA in *Calca*^{-/-}-deficient mice after 10 mg/kg LPS i.p (n=4). *p<0.05 indicates statistically significant differences compared to controls, #p<0.05 indicates statistically significant differences between treated groups.
- Fig. 8: shows the proteome profiling of serum cytokines in *αCgrp-*deficient mice 4 and 10 h after 10 mg/kg LPS ip (pooled serum of n=4 per group and per time point).
- Fig. 9: shows the expression of *II1b* and *II17a* in selected organs 24 h after sham operation or CLP in WT mice, as indicated (n=4). *p<0.05 indicates statistically significant differences.

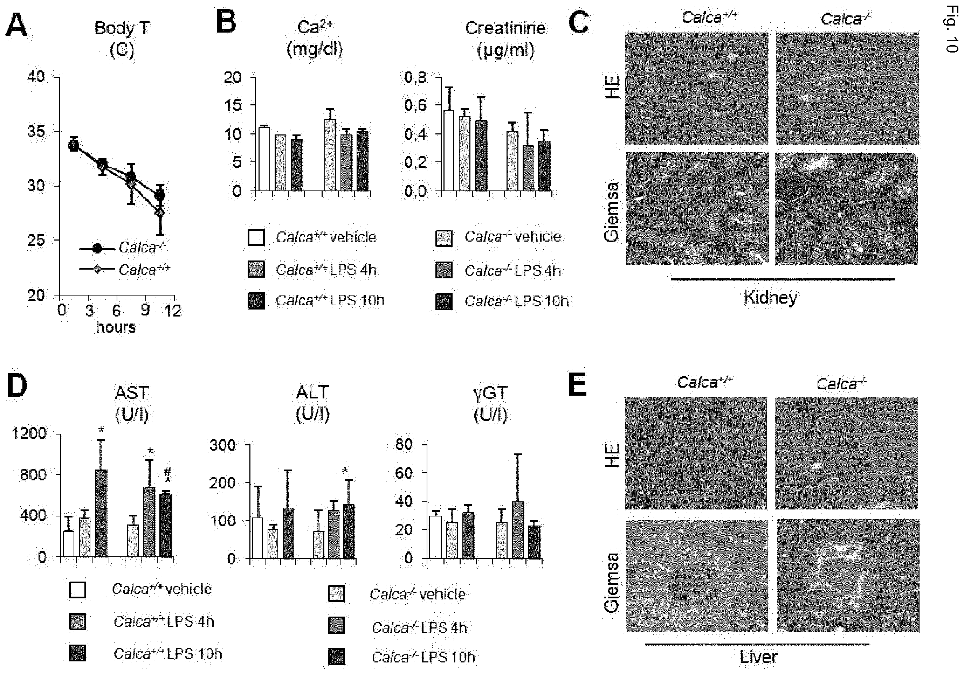

- Fig. 10: shows **A)** Rectal body temperature in *Ca*/*ca*-deficient mice and controls at the indicated time points following 10 mg/kg LPS ip (n=4). **B)** Total calcium and creatinine levels in mice of the same genotypes at the indicated time points (n=4). **C)** Representative Masson's trichrome and HE staining of kidney samples 24 h after 10 mg/kg LPS i.p in mice of the indicated genotypes. **D)** Liver function parameters in *Calca*^{+/+} and *Calca*^{-/-} mice 0, 4, and 10 h after 10 mg/kg LPS ip (n=4). **E)** Representative Masson's trichrome and HE staining of liver samples 24 h after 10 mg/kg LPS ip in mice of the indicated genotypes. *p<0.05 indicates statistically significant differences compared to controls, #p<0.05 indicates statistically significant differences between treated groups.
- Fig. 11: shows proteome profiling of serum cytokines in calcitonin receptor- (*Calcr*) deficient mice 4 and 10 h after 10 mg/kg LPS ip (pooled serum of n=4 per group and per time point).
- Fig. 12: shows survival of WT mice receiving a single intraperitoneal injection of either vehicle or olcegepant (100 mg/kg) 4h after 40 mg/kg LPS ip (n=8-10 mice per group).
- Fig. 13: shows the suggested model of PCT action during experimental septic shock (MP=macrophages, DC=dendritic cells).

### Examples

Procalcitonin (PCT) represents the clinically most widely used biomarker of sepsis. To date, its pathophysiological function remains unclear. The present invention shows herein, that PCT-deficient mice (*Calca*^{*-*/*-*}) display increased survival following cecal ligation and puncture or intraperitoneal injections of lipopolysaccharide. Expression analysis revealed decreased II1b, II6 and II17a levels in addition to reduced macrophage influx in lungs of *Calca*^{-/-}-mice with systemic inflammation, coinciding with predominant pulmonary expression of the putative PCT receptor CRLR. PCT stimulated proinflammatory cytokine expression in macrophages and dendritic cells, including *II1b* and *II6*, required for *II17a* expression in γδT cells. In septic polytrauma patients, PCT positively correlated with systemic IL17A. In turn, pharmacologic neutralization of II17a or treatment with olcegepant, a specific CRLR antagonist developed for migraine treatment, inhibited the deleterious effect of PCT in septic mice. The present invention provides evidence for a mediator role of PCT in sepsis with immediate therapeutic potential for affected patients.

### Calca controls survival in septic shock

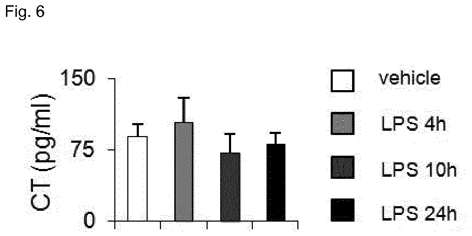

In order to clarify the current incomprehension, the inventors analyzed the role of PCT in lethal systemic inflammation using two established mouse models of septic shock. As it was previously reported that mice fail to increase PCT levels during severe infection, they first monitored *Calca* expression in WT mice subjected to aggressive cecal ligation and puncture (CLP), causing lethal polymicrobial sepsis. Similar to what can be observed in humans, an increased expression of *Calca* was detected in a variety of analyzed tissues 24h after CLP in WT mice (Figure 1A). To investigate whether these findings can be replicated in another model of septic shock, WT mice were challenged with intraperitoneal injections of LPS, characterized by sterile endotoxemia with a more rapid and temporary rise in inflammatory mediators compared to CLP, and detected a similar increase in *Calca* expression in analyzed tissues after 4 and 10h (Figure 1 B). Since *Calca* not only encodes PCT, but also Calcitonin (CT) and Calcitonin gene-related peptide alpha (αCgrp), serum levels of the respective peptides were monitored next, and elevated serum levels of PCT and αCgrp were found after LPS challenge (Figure 1C). In contrast, CT levels remained unchanged (Figure 6). As these findings demonstrated a robust upregulation of *Calca* in both sepsis models, the effects of *Calca*-deficiency were studied on outcome in septic shock. Log-rank analyses revealed improved survival of *Calca*-deficient mice following CLP (Figure 1 D), and the same was observed in mutant mice receiving a lethal dose of LPS ip (Figure 1 E). In contrast, αCgrp-deficient mice were not protected from lethal endotoxemia (Figure 1 F), indicating that the survival benefit observed in *Calca*-deficient mice is caused by a deleterious effect of PCT.

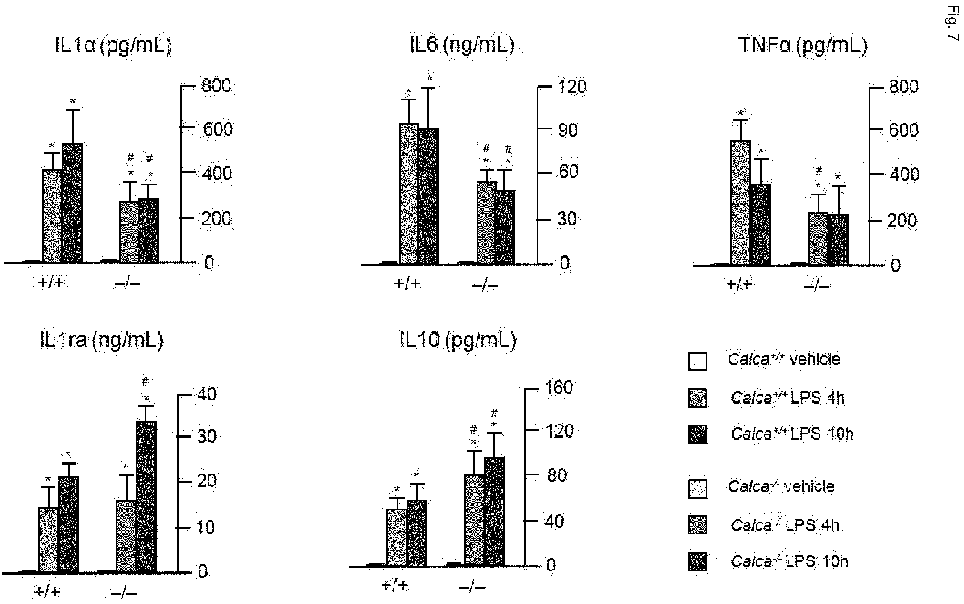

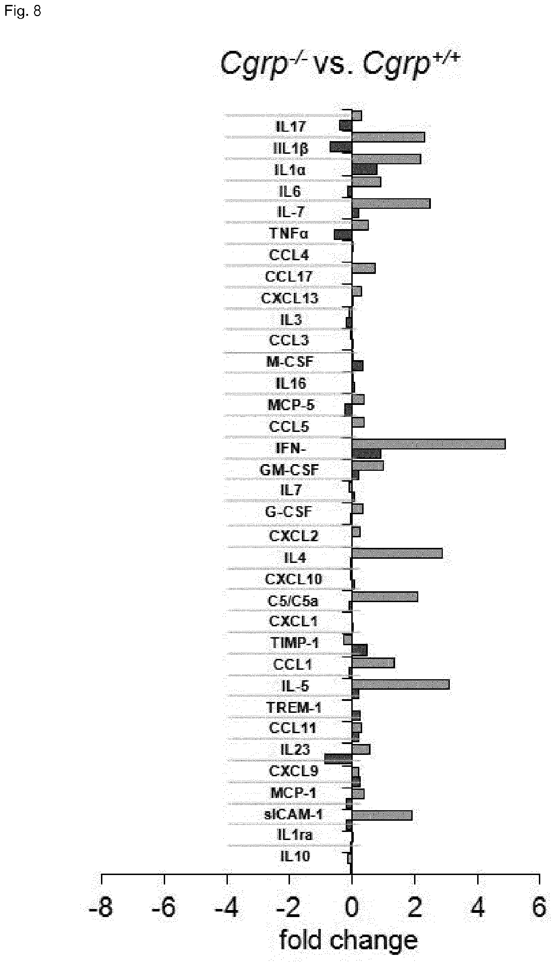

To understand these observations on the molecular level, the effects of *Calca*-deficiency were assessed on systemic cytokine concentrations following endotoxemia. Proteome profiling and confirmation by ELISA revealed decreased concentrations of pro-inflammatory cytokines including Interleukin-1 alpha (II1a) and Interleukin-6 (II6) in addition to increased levels of anti-inflammatory interleukin-10 (II10) and interleukin-1 receptor antagonist (II1ra) in *Ca*/*ca*-deficient mice (Figure 1G and H, Figure 7). The most pronounced differences, however, were detected in the case of Interleukin-I beta (II1b) and Interleukin-17a (II17a), whose serum levels were significantly lower in *Calca*-deficient mice compared to WT controls. In contrast, no consistent alterations in serum cytokine levels could be detected in *αCgrp-*deficient mice (Figure 8, Figure 1 H). In order to rule out an intrinsic cell-autonomous defect in II1b or II17a synthesis, the inventors challenged *Ca*/*ca*-deficient mice with LPS ip and/or PCT ip. While an effect alone was undetectable, PCT co-administered with LPS augmented the increase in systemic II1b and II17a levels in turn (Figure 1I).

### Calca regulates pulmonary macrophage influx

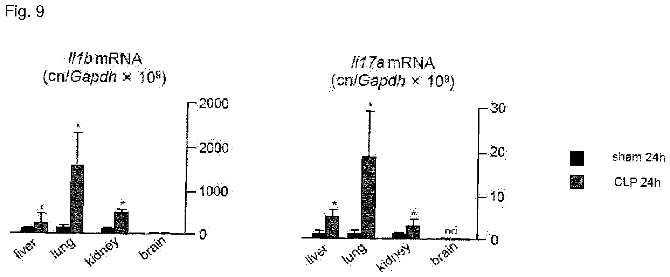

Characterizing gene expression in tissue extracts, it was found that *II1b* and *II17a* were predominantly expressed in pulmonary and white adipose tissue after LPS challenge (Figure 2A) and CLP (Figure 9). Compared to WT controls, reduced levels of both *II1b* and *II17a* were detected in lung and white fat from *Calca*-deficient mice. Previous studies demonstrated that PCT primarily binds to the calcitonin receptor-like receptor (*Calcrl*), whereas weak agonistic activity was also observed at calcitonin receptor (CTR). Monitoring the expression of putative PCT receptors, high and specific expression of *Calcrl* was found in lung tissue, subsequently decreasing during septic shock (Figure 2B). In contrast, *Calcr* expression, encoding CTR, was detected in lung, kidney and fat, exhibiting less pronounced kinetics in the course of sepsis progression.

As no difference in kidney and liver function was observed (Figure 10), and protein analyses confirmed reduced concentrations of II1b and II17a in flushed lungs of *Calca*-deficient mice (Figure 2C), these results indicated a role of PCT in regulating pulmonary integrity during septic shock. This observation coincided with the fact that acute respiratory distress syndrome (ARDS) represents one of the clinically most challenging organ dysfunction in patients with septic shock. II1b and II17a were previously shown to regulate macrophage activation and recruitment. FACS analysis of lung digests demonstrated elevated numbers of granulocytes, while reduced populations of lymphocytes, natural killer and B-cells were observed in *Ca*/*ca*-deficient mice (Figure 2D). Notably, significantly decreased numbers of macrophages and dendritic cells (DC) were found in mutant animals, accompanied by elevated γδT cells. These results were confirmed by immunohistochemistry, where decreased numbers of pulmonary intravascular CD68 positive macrophages were observed (Figure 2E). Moreover, a reduced expression of intercellular adhesion molecule 1 (*Icam1*) and E-selectin (*Sele*), two crucial mediators of macrophage adhesion and transmigration, was detected in lungs of *Ca*/*ca*-deficient mice (Figure 2F).

### PCT orchestrates expression of LPS-induced cytokines

Monitoring the expression of *Calca* in a variety of different cell types, *ex vivo* macrophages and DC cultures were identified to display high *Calca* expression after stimulation with LPS, whereas primary adipocyte and osteoblast cultures, known to contain a small fraction of macrophages showed only moderate expression (Figure 3A). Likewise, apart from adipocytes, macrophages and DC were found to express high levels of one of the two putative PCT receptors, CRLR (Figure 3B). Upon stimulation with LPS, evoking robust *Calca* expression in WT macrophages, a reduced expression of proinflammatory cytokines including *II6*, *II1b,* and *II1a* was detected in *Ca*/*ca*-deficient macrophages, whereas an increase was observed in the case of *II10* and *II1ra* (Figure 3C,D). To differentiate between a cell-autonomous defect and an autocrine role of macrophage-derived PCT, macrophages were stimulated with LPS, PCT, or both. In WT macrophages, selective LPS or PCT treatment caused an increase in *Calca*, while the combined administration resulted in a potentiation of *Calca* expression, indicating a potent auto-stimulatory effect of PCT in macrophages (Figure 3E). While PCT alone specifically induced the expression of *II6*, it potentiated the stimulatory effect of LPS on *II6*, *II1b,* and *II1a*, and inhibited *II10* and *II1ra* expression in *Calca*^{-/-} macrophages (Figure 3F,G).

### PCT indirectly controls inflammation through II17a

As stimulation with PCT, LPS, or both failed to induce a detectable expression of *II17a* in primary macrophages (data not shown), the differential regulation of pulmonary *II17a* in *Ca*/*ca*-deficient mice remained unclear. Previously it was shown that DC function as crucial regulators of γδT cells, representing a specific subset of T cells and the primary source of II17a in experimental sepsis. In contrast to *ex vivo* generated macrophages, PCT did not augment the stimulatory effect of LPS on *Calca* expression in cultured DC (Figure 4A). Conditioned medium from WT DC stimulated with LPS evoked a powerful II17a response in WT yδT cells on mRNA and protein level, which was blunted upon stimulation with conditioned medium from *Ca*/*ca*-deficient DC (Figure 4B). Notably, this effect was specific for II17a, as other cytokines synthesized in γδT cells including *II6* and *II1b* were expressed at similar levels (Figure 4C). In agreement with only weak expression of *Calcrl* in γδT cells, a direct effect of PCT on γδT cells could be excluded as neither PCT alone nor conditioned medium from *Ca*/*ca*-deficient DC substituted with PCT affected *II17a* expression (Figure 4D). Searching for an explanation, the expression of cytokines essential for II17a synthesis was monitored in stimulated DC. Whereas the induction of *II6* and *II23* was not affected, PCT caused an augmentation of *II1b* expression upon co-stimulation with LPS, essentially required for II17a synthesis in γδT cells (Figure 4E). Together, these observations indicated that PCT links the innate and adaptive immune system and controls harmful inflammatory responses through II17a *in vitro.*

In order to test whether II17a acts downstream of PCT *in vivo, Calca*-deficient mice were treated with a lethal dose of LPS, followed by PCT and/or an II17a neutralizing antibody previously shown to protect mice from septic shock. PCT abrogated the survival benefit of *Calca*-deficiency in lethal endotoxemia (Figure 4F). In turn, neutralization of II17a blunted the direct negative effect of concomitant PCT. Next, the inventors monitored a possible association between PCT and II17A in humans and assessed a group of polytrauma patients who subsequently developed PCT-positive sepsis. In this cohort a time-dependent increase in PCT and IL17A levels was observed (Figure 4G). Although limited by a small sample size, PCT levels at 96h after admission positively correlated with ventilation days on the intensive care unit, supporting the fact that PCT exerts a negative effect on pulmonary integrity (Figure 4H). No association of C-reactive protein and leukocytes was detected, whereas IL17A positively correlated with PCT at 48, 72, and 96h (Figure 4I).

### Regulation of mortality in sepsis through PCT-CRLR signaling

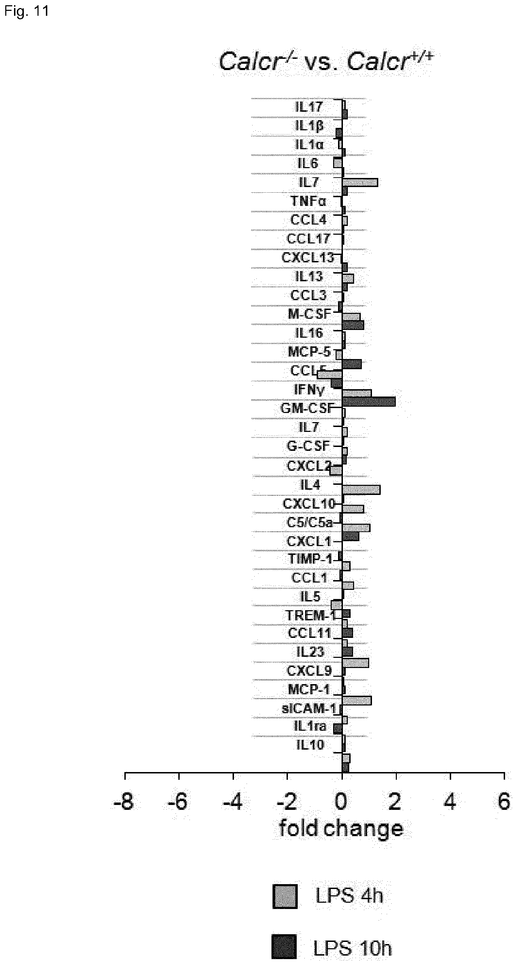

PCT was previously shown to be able to signal through both CTR and CRLR *in vitro.* In order to identify the biologically relevant PCT receptor, mice lacking CTR were subjected to LPS ip. CTR-deficient mice displayed no major alteration in systemic cytokine response including II1β and II17A and exhibited normal survival in lethal endotoxemia compared to WT littermates (Figure 5A,B; Figure 11). These findings excluded a pivotal role of CTR signalling in systemic inflammation and pointed towards CRLR as the relevant PCT receptor. Therefore, the inventors next explored the effects of pharmacologically inhibiting CRLR signalling through employment of olcegepant (BIBN4096), a highly specific, small molecule CRLR antagonist initially developed for migraine treatment. Olcegepant inhibited the LPS-dependent stimulatory effect of PCT on II1b and II6 expression in macrophages *in vitro* (Figure 5C). Furthermore, olcegepant ip led to a significant reduction of II1b and II17a levels in mice following LPS challenge *in vivo* (Figure 5D).

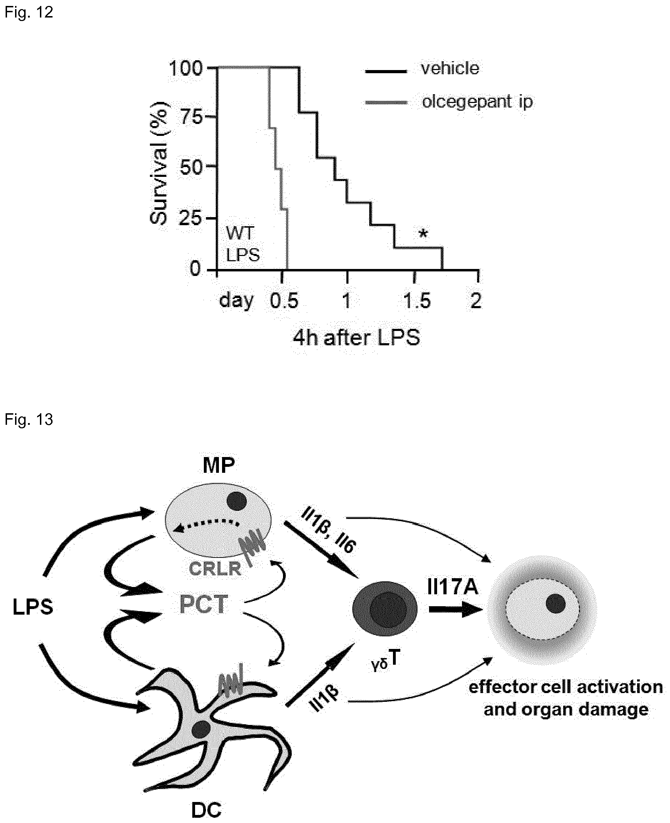

As these results suggested a therapeutic potential of inhibiting PCT signaling at CRLR, olcegepant ip was applied immediately before LPS, resulting in a significant reduction of mortality in WT mice (Figure 5E). Likewise, mortality was significantly reduced in WT mice subjected to CLP receiving subcutaneous olcegepant injections every 12h (Figure 5F). Mechanistically, olcegepant co-administered with LPS ip did not further improve the survival in *Ca*/*ca*-deficient mice (Figure 5G). CRLR also mediates the biologic effects of αCgrp, however olcegepant significantly improved mortality rates in *αCgrp-*deficient animals (Figure 5H). This demonstrated that systemic CRLR antagonism increases survival through inhibition of PCT signaling, independent of αCgrp. For preclinical testing, olcegepant ip was administered 4h after injections of LPS which resulted in an increased mortality, suggesting that CRLR antagonism in the inflamed peritoneal cavity results in a severe deterioration of local inflammation, possibly through αCgrp blockade (Figure 12). As in this case, local inflammatory processes could have potentially caused insufficient systemic uptake of ip olcegepant, olcegepant was applied subcutaneously 4h after ip LPS challenge. While a trend towards improved survival was observed with a single subcutaneous injection, intermittent treatment every 12 hours significantly prolonged survival (Figure 5I). Moreover, the favorable effect of olcegepant was also observed when it was subcutaneously administered to WT mice 4h before induction of lethal endotoxemia (Figure 5J). These experiments underline the therapeutic and prophylactic potentials of olcegepant, as most affected patients receive delayed treatment, or present with an increased risk to develop sepsis due to conditions associated with misregulated systemic inflammation such as polytrauma or surgery (Singer 2016).

The results presented herein provide evidence for a role of PCT in modulating LPS-induced cytokine response in antigen-presenting cells, including II1b and II6. This favours release of II17a from γδT cells and results in further macrophage influx and tissue destruction during septic shock (Figure 13). The results indicate that II1b and, in particular, II17a function as distal effectors of PCT, linking the innate to the adaptive immune system in septic shock. A recent study employing mice lacking *Calca* failed to detect an influence on the outcome of severe infection, however no increase in *Calca* expression or PCT levels was detected. Thus, two aggressive models of lethal systemic inflammation were utilized, exhibiting a mortality rate of 100% in WT mice, and a marked increase in *Calca* mRNA and PCT levels was detected. A possible contribution of PCT cleavage products such as N- and C-terminal PCT to the observed effects was not specifically tested and must not be excluded, as local or systemic proteolytic processing even of pharmacologically administered PCT is theoretically possible. Despite these limitations, the results demonstrate that PCT represents the only relevant factor among *Calca*-derived peptides determining outcome in experimental septic shock. This is unexpected, as αCgrp was previously shown to reduce mortality in animal models of sepsis (Gomes et al., Calcitonin gene-related peptide inhibits local acute inflammation and protects mice against lethal endotoxemia, Shock. 2005 Dec;24(6):590-4; Lee et al., Deficiency of alpha-calcitonin gene-related peptide induces inflammatory responses and lethality in sepsis. Cytokine. 2013 Nov;64(2):548-54). The fact that PCT seems to override αCgrp signalling through the same receptor, CRLR, is especially interesting from a clinical point of view, as up to now pharmacologic blockade of CRLR was considered a counterproductive therapeutic approach in systemic inflammation. A potential role of αCgrp in regulating local inflammation is supported by the observation, that administration of olcegepant into the inflamed peritoneal cavity resulted in decreased survival. Thus, blockade of CRLR signalling must occur systemically, and not locally at the site of infection, in order to improve outcomes in states of lethal systemic inflammation.

PCT appears to function as a modulator in septic shock rather than a pivotal local effector cytokine, providing a potential advantage over past immunomodulatory agents failing to improve outcomes in sepsis. As such, olcegepant and its related compounds, having recently been tested vigorously in humans suffering from migraine, could exhibit a tolerable safety profile in the context of life-threatening conditions such as septic shock. Furthermore, CRLR blocking antibodies, with even less adverse effects, are currently being developed (Edvinsson, CGRP receptor antagonists and antibodies against CGRP and its receptor in migraine treatment. Br J Clin Pharmacol. 2015 Aug;80(2):193-9). In summary, the present invention reveals an unexpected approach to advance therapeutic strategies for patients with systemic inflammation.

### Methods and Materials

### Mice

*Calca*-*, Calcr-,* and *Cgrp*-deficient mice were genotyped as described previously (Lu, J.T., et al. Mice lacking alpha-calcitonin gene-related peptide exhibit normal cardiovascular regulation and neuromuscular development. Mol Cell Neurosci 14, 99-120 (1999), Hoff, A.O., et al. Increased bone mass is an unexpected phenotype associated with deletion of the calcitonin gene. J Clin Invest 110, 1849-1857 (2002), Keller, J., et al. Calcitonin controls bone formation by inhibiting the release of sphingosine 1-phosphate from osteoclasts. Nat Commun 5, 5215 (2014). CLP was performed as described previously (Rittirsch, D., Huber-Lang, M.S., Flierl, M.A. & Ward, P.A. Immunodesign of experimental sepsis by cecal ligation and puncture. Nat Protoc 4, 31-36 (2009). Briefly, an approximately 1 cm abdominal midline incision was made, followed by exposure of the cecum. After ligation, the cecum was punctured three times through and through with a 19-gauge needle and a small portion of feces was pressed out. After repositioning of the bowel, the abdomen was closed in layers using surgical sutures and metallic clips, and 1 ml of 0.9% NaCl (37° Celsius) was slowly injected subcutaneously for resuscitation. For induction of lethal endotoxemia, mice received an intraperitoneal (ip) injection of 40 mg/kg LPS. Survival was monitored at least every 6 h for 7 days after which the experiments were terminated. For pharmacologic proof-of-principle experiments involving II17a, mice received 100 µg/kg PCT and/or 2.5 µg/kg antibody treatment ip 30 minutes before and 12h after induction of septic shock through 40 mg/kg LPS ip. Non-specific IgG was used at equal concentrations. Olcegepant (100 mg/kg) was administered intraperitoneally or subcutaneously as indicated. For *in vivo* experiments involving expression, histological, and FACS analyses, mice were challenged with a sublethal dose of LPS (10 mg/kg ip). The use of *in vivo* experiments, especially CLP, was kept to a minimum throughout the study (replace, reduce, refine) in accordance to current recommendations for animal welfare in sepsis research (Lilley 2015). Experimental procedures were performed with approval from the animal care committees of the University Medical Center Hamburg-Eppendorf and the *Behörde für Umwelt und Gesundheit der Hansestadt Hamburg.*

### Peptides and antibodies

LPS (E. coli serotype O111:B4) was derived from Sigma Aldrich. Recombinant human PCT was obtained from Prospec, as commercially available recombinant mouse PCT exhibited no biological activity in any assay tested. GM-CSF and Interleukin-4 were obtained from R&D (#415-ML-050, #404-ML-050). A monoclonal antibody specifically neutralizing II17a was obtained from eBioscience (#16-7173-81). Non-specific IgG was obtained from Jackson ImmunoResearch. Olcegepant (BIBN4096S) was obtained from Tocris.

### Serum analyses

Serum was obtained by cardiac puncture. Proteome profiling was performed using Mouse Cytokine Panel A (R&D, #ARY006). Confirmation experiments for II1b, II17, II1a, II6, TNFa, II1ra, II10 were performed using ELISA (R&D, #PMLB00C, #M1700, #MLA00, #M6000B, #MTA00B, #MRA00, #M1000B). Serum concentrations of PCT, CT and CGRP were measured by ELISA (Kamiya #KT-25866, Bachem #S-1488.0001 BA, S-1167.0001 BA).

### Expression analyses

RNA was isolated using the RNeasyMini kit (Qiagen). For qRT-PCR expression analysis, 1 µg of RNA was reverse transcribed using SuperScriptIII (Invitrogen) according to the manufacturer's instructions. qRT-PCR analysis was performed using predesigned TaqMan gene expression assays (Applied Biosystems) and Gapdh as internal control.

### Flow cytometric analyses.

For flow cytometry analyses, animals were euthanized and perfused with phosphate buffered saline (PBS). After dissection, lungs were enzymatically digested in collagenase solution and passed through a 40µm cell strainer to remove cell debris. The resulting single cell suspensions were incubated with the appropriate antibody cocktails followed by analyses with a LSR Fortessa flow cytometer (BD Pharmingen, Franklin Lakes, NJ, USA) and FACS Diva software (Version 6.2, BD Pharmingen, Franklin Lakes, NJ, USA). The antibodies used in this study are listed in the antibody reference list in the supplemental methods section.

### Histology

After sacrifice, lung tissues were fixed in 3.7% PBS-buffered formaldehyde, dehydrated in ascending concentrations of ethanol, and embedded in paraffin. Sections of 4µm thickness were cut, deparaffinized, hydrated, and stained with haematoxylin-eosin or Giemsa dye solution by using standard protocols.

### Immunhistochemistry

4 µm FFPE lung sections were de-paraffinized, pre-treated with DAKO S1699 antigen retrieval solution, pH6, 2x4' in a microwave, blocked with normal rabbit serum and mouse FcR blocking reagent, incubated for 1h at room temperature with rat-anti-mCD68 (abcam #53444), washed and incubated with biotinylated rabbit-anti-rat secondary antibody for 30'. After washing, the secondary antibody was detected using the streptavidin-alkaline phosphatase kit (ABC-AP, Vector Labs.). Enzyme reactivity of the alkaline phosphatase complex was visualized using DAKO liquid permanent red.

### Cell culture

Primary macrophages were generated from bone marrow cells cultured in DMEM supplemented 10% FCS and 40 ng/ml M-CSF for 7 days. Primary DC were generated from mouse bone marrow cells which were grown in RPMI 1640 medium with 10% FBS supplemented with 100 U/ml GM-CSF and 20 ng/ml IL-4 for 7 days. For γδT cell preparations, spleens were dissected and processed through a 40-µm filter, followed by lysis of erythrocytes. γδT lymphocytes were subsequently purified using magnetic labeled beads (Miltenyi Biotec, #130-092-125). For the generation of primary osteoblasts, bone marrow cells were differentiated for 10 days in the presence of 25 µg/ml ascorbic acid and 5 mM β-glycerophosphate as described previously (Keller, J., et al. Calcitonin controls bone formation by inhibiting the release of sphingosine 1-phosphate from osteoclasts. Nat Commun 5, 5215 (2014)). Mouse embryonic fibroblasts were cultured in DMEM supplemented with 10% FCS as described previously (Marschner, K., Kollmann, K., Schweizer, M., Braulke, T. & Pohl, S. A key enzyme in the biogenesis of lysosomes is a protease that regulates cholesterol metabolism. Science 333, 87-90 (2011)). For primary adipocytes, epididymal fat pads were digested using collagenase. The adipose cells were washed several times and incubated with Dulbecco's modified Eagle's medium containing 5% bovine serum albumin. Primary hepatocytes were harvested as described and cells seeded in DMEM containing 10% FCS to a density of 200,000 cells per well in collagen-coated 12 well plates. Fully differentiated cells were stimulated with 10 µg/ml LPS and/or 200 ng/ml PCT, as indicated.

### Co-culture system

After 7 days of maturation, 10⁵ DC were plated in 12-well tissue culture plates and stimulated with 10 µg/ml LPS for 24 h. DC conditioned medium was then transferred onto γδT cells. In some experiments, conditioned medium was supplemented with 200 ng/ml PCT 200 ng/ml PCT. After 24 h, samples were spun, and the supernatant was assayed for IL-17. For expression analyses, mRNA was extracted from cell pellets using RNeasyMini kit (Qiagen) according to standard procedures.

### Human samples

For assessment of human samples PCT and IL17A levels were retrospectively analyzed in a previously characterized group of patients. In this observational pilot study, 20 patients with polytrauma und subsequent sepsis were included between April 2009 and March 2012. Inclusion criteria were polytrauma, age of 18 years or more, ISS of greater than 25 points, admission less than 2 hours and transfer less than 6 hours after trauma with a therapy-free interval of less than 30 minutes, in addition to developing PCT-positive sepsis within the first 96h of admission (34%). The study cohort consisted of patients with severe polytrauma (mean ± SD: injury severity score, 43 ± 13 points; polytrauma score, 52 ± 20 points), which was reflected by a high mortality rate (n = 25%). Among non-survivors, mean survival time was 9.4 ± 7 days. The sepsis origin was wound infection in 50%, pneumonia in 27%, and catheter-associated infection in 23%. PCT, leukocyte number (white blood count, WBC) and C-reactive protein (CRP) levels were determined in the respective laboratory of the University Hospital Charité, Campus Virchow-Klinikum, according to standard procedures. IL17A levels were measured using a conventional ELISA (Thermo Scientific). Measurements were approved by the Landesamt fur Gesundheit und Soziales, Berlin.

### Statistical analyses

The number of samples was chosen according to established standards providing sufficient statistical power to enable the detection of biologically relevant differences in animal models. Data were analyzed by two-tailed Student's t-test using Excel software. In the case of multiple comparisons, Bonferroni correction was used. Survival analyses were performed using the log-rank-test. If not indicated otherwise, all data are reported as mean ± SD. p<0.05 was considered statistically significant.

Antibodies used in the present invention are listed in the antibody reference list below.

| **Primary antibody** | **Donor species** | **Isotype** | **Dilution Catalog number supplier** |
|---|---|---|---|
| CD11 b-PE_CF594 clone M1/70 | Rat | Clone | 1:300 |
| | | M1/70, | 562287 |
| | | IgG2b, K | BD Pharmingen, Franklin Lakes, NJ, USA |
| Ly-6G-Alexa700 clone 1A8 | Rat | Clone | 1:100 |
| | | 1A8, | 561236 |
| | | IgG2a, K | BD Pharmingen, Franklin Lakes, NJ, USA |
| CD45-APC-CY7 | Rat | Clone | 1:100 |
| | | 30-F 11 | 561037 |
| | | IgG2b, K | BD Pharmingen, Franklin Lakes, NJ, USA |
| CD8a-BV650 | Rat | Clone | 1:100 |
| | | 53-6.7 | 100741 |
| | | IgG2a, K | Biolegend |
| | | | San Diego, CA, USA |
| CD4-BV605 | Rat | Clone | 1:100 |
| | | RM4-5 | 100537 |
| | | IgG2a, K | Biolegend San Diego, CA, USA |
| CD3e-V500 | Syrian hamster | Clone | 1:100 |
| | | 500A2 | 560771 |
| | | IgG2, K | BD Pharmingen, Franklin Lakes, NJ, USA |
| TCR γ/δ BV422 | Armenian hamster | Clone | 1:100 |
| | | GL3 | 118119 |
| | | IgG | Biolegend |
| | | | San Diego, CA, USA |
| Ly-6C PerCP-Cy5.5 | Rat | Clone | 1:50 |
| | | HK1.4 | 45-5932-80 |
| | | IgG2c | eBioscience, San Diego, USA |
| CD11c-488 | Armenian hamster | Clone | 1:100 |
| | | N418 | 53-0114-80 |
| | | IgG | eBioscience, San Diego, USA |
| Nk1.1 PE-Cy7 | Mouse | Clone | 1:100 |
| | | PK136 | 561117 |
| | | IgG2a,k | BD Pharmingen, Franklin Lakes, NJ, USA |
| B220- PE-Cy5.5 | Rat | Clone | 1:100 |
| | | RA3-6B2 | RM2618 |
| | | IgG2a,k | Thermo Fisher Scientific Waltham, MA U.S.A. |

## Claims

1. Compound for use in a method for preventing or treating a procalcitonin mediated systemic inflammation associated disease, wherein said compound is an inhibitor or antagonist of CRLR, RAMP1, RAMP2, RAMP3 or RCP.

2. The compound for use in a method for preventing or treating a procalcitonin mediated systemic inflammation associated disease according to claim 1, wherein said procalcitonin mediated systemic inflammation associated disease is sepsis.

3. The compound for use in a method for preventing or treating a procalcitonin mediated systemic inflammation associated disease according to claim 1 or 2, wherein said compound is selected from
- N-((1*R*)-2-(((1*S*)-5-amino-1-((4-(pyridin-4-yl)piperazin-1-yl)carbonyl)pentyl)amino)-1-(3,5-dibromo-4-hydroxybenzyl)-2-oxoethyl)-4-(2-oxo-1,4-dihydroquinazolin-3(2H)-yl)piperidine-1-carboxamide,
- N-((3*R*,6*S*)-6-(2,3-difluorophenyl)-2-oxo-1-(2,2,2-trifluoroethyl)hexahydro-1H-azepin-3-yl)-4-(2-oxo-2,3-dihydro-1H-imidazo[4,5-b]pyridin-1-yl)piperidine-1-carboxamide,
- 2-((8*R*)-8-(3,5-difluorophenyl)-10-oxo-6,9-diazaspiro(4.5)dec-9-yl)-N-((2R)-2'-oxo-1,1',2',3-tetrahydrospiro(indene-2,3'-pyrrolo[2,3-b]pyridin)-5-yl)acetamide;
- a hydroxy pyridin carboxamide CRLR antagonist;
- an oxepino[3,4-e]indazol-8-one CRLR antagonist;
- an azepino[3,4-e]indazol-8-one CRLR antagonist;
- an azepine CRLR antagonist;
- GPRG₈₋₃₇,
- a ligand specifically reactive to any one of CRLR, RAMP1, RAMP2, RAMP3 or RCP, or
- a modulator of the expression of a gene encoding any one of CRLR, RAMP1, RAMP2, RAMP3 or RCP.

4. The compound for use in a method for preventing or treating a procalcitonin mediated systemic inflammation associated disease according to claim 3, wherein said ligand specifically reactive to any one of CRLR, RAMP1, RAMP2, RAMP3 or RCP is an antibody.

5. The compound for use in a method for preventing or treating a procalcitonin mediated systemic inflammation associated disease according to claim 3, wherein said modulator of the expression of a gene encoding CRLR, RAMP1, RAMP2, RAMP3 or RCP is a nucleic acid molecule or a precursor thereof comprising a sequence tract complementary to an mRNA molecule encoding any one of CRLR, RAMP1, RAMP2, RAMP3 or RCP.

6. The compound for use in a method for preventing or treating a procalcitonin mediated systemic inflammation associated disease according to any one of the preceding claims, wherein said compound is systemically or locally administered provided that said compound is not locally administered at a local inflamed or infected site.

7. A pharmaceutical composition for use in a method for preventing or treating a procalcitonin mediated systemic inflammation associated disease, particularly sepsis, comprising a compound according to any one of claims 1 to 5, and optionally a pharmaceutical acceptable carrier and/or excipient.

8. The pharmaceutical composition for use in a method for preventing or treating a procalcitonin mediated systemic inflammation associated disease according to claim 7 formulated for epicutaneous, subcutaneous, intramuscular, intravenous, intraarterial, intraperitoneal, intraspinal, intracerebral, peridural, intradural, intrapulmonal, rectal, vaginal, nasal, oral or sublingual application.

9. A dosage form for use in a method for preventing treating a procalcitonin mediated systemic inflammation associated disease, particularly sepsis, comprising a compound according to any one of claims 1 to 5.

10. The dosage from for use in a method for preventing or treating a procalcitonin mediated disease according to claim 9, wherein said compound is formulated as an oral formulation, nasal inhalant, injection form, suppository or topical formulation.

11. A method of manufacturing a medicament for preventing or treating a procalcitonin mediated systemic inflammation associated disease, particularly sepsis, comprising the use of a compound according to any one of claims 1 to 5.

12. A method for preventing or treating a procalcitonin mediated systemic inflammation associated disease, particularly sepsis, comprising administering a compound according to any one claims 1 to 6 to a patient in need thereof, particularly said compound in a pharmaceutically effective amount.
